Europäisches Patentamt
European Patent Office
Office européen des brevets

(19) (11) Veröffentlichungsnummer: **0 000 393 A1**

(12) # EUROPÄISCHE PATENTANMELDUNG

(21) Anmeldenummer: **78100356.1**

(22) Anmeldetag: **11.07.78**

(51) Int. Cl.²: **C07D501/20,** A61K31/545, A23K1/17

(30) Priorität: **16.07.77 DE 2732323**

(43) Veröffentlichungstag der Anmeldung: **24.01.79 Patentblatt 79/2**

(84) Benannte Vertragsstaaten: **BE CH DE FR GB LU NL SE**

(71) Anmelder: **Bayer Aktiengesellschaft, Zentralbereich Patente,Marken und Lizenzen Bayerwerk, D-5090 Leverkusen 1 (DE)**

(72) Erfinder: **Preiss, Michael, Dr., Egenstrasse 21, D-5600 Wuppertal 1 (DE)**
**König, Hans-Bodo, Dr., Herberts Katernberg 10, D-5600 Wuppertal 1 (DE)**
**Metzger, Karl Georg, Dr., Pahlekestrasse 15, D-5600 Wuppertal 1 (DE)**

(54) **Beta-Lactam-Verbindungen, Verfahren zu ihrer Herstellung sowie ihre Verwendung.**

(57) β-Lactam-Verbindungen der Formel

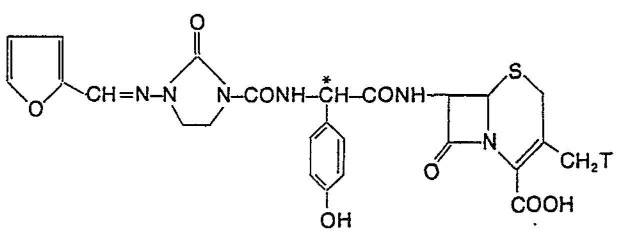

in der
T Wasserstoff, Alkyl–CO–O–, Pyridinium, 4-Carbamoylpyridinium, Aminopyridinium, Carbamoyloxy, Azido, Cyano, Hydroxy, die Gruppe –S–Phenyl, welche substituiert sein kann, oder die Gruppe –S–Het bedeutet, in welcher Het für einen gegebenenfalls substituierten heterocyclischen 5- oder 6gliedrigen Ring steht;
ein Verfahren zu ihrer Herstellung sowie deren Verwendung als Arzneimittel, insbesondere als antibakterielle Mittel und als Mittel zur Förderung des Wachstums und zur Verbesserung der Futterverwertung bei Tieren.

EP 0 000 393 A1

BAYER AKTIENGESELLSCHAFT
Zentralbereich
Patente, Marken und Lizenzen

5090 Leverkusen, Bayerwerk
PG/AB

ß-Lactam-Verbindungen, Verfahren zu ihrer Herstellung sowie ihre Verwendung

Die vorliegende Erfindung betrifft neue ß-Lactam-Verbindungen, ein Verfahren zu ihrer Herstellung sowie ihre Verwendung als Arzneimittel, insbesondere als antibakterielle Mittel und als Mittel zur Förderung des Wachstums und zur Verbesserung der Futterverwertung bei Tieren.

Es ist bereits bekannt geworden, daß bestimmte ℒ-(3-Hetarylidenamino-2-oxo-imidazolidin-1-yl)-carbonylamino)-benzylpenicilline antibakteriell wirksam sind (vgl. Deutsche Offenlegungsschrift 2 525 541).

Es wurde gefunden, daß die neuen ß-Lactam-Verbindungen der Formel I

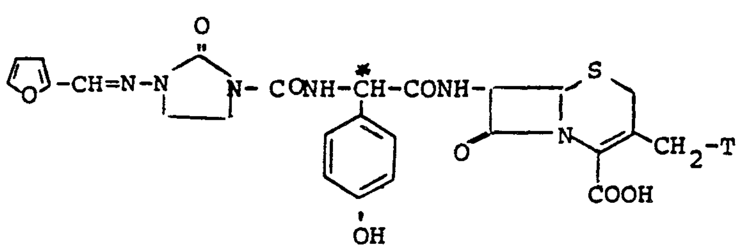

(I)

in der

T Wasserstoff, Alkyl-CO-O-, Pyridinium, 4-Carbamoylpyridinium, Aminopyridinium, Carbamoyloxy, Azido, Cyano, Hydroxy, die Gruppe -S-Phenyl, welche substituiert sein kann, oder die Gruppe -S-Het bedeutet, in welcher Het für einen gegebenenfalls substituierten heterocyclischen 5- oder 6-gliedrigen Ring steht;

wobei diese Verbindungen der Formel I bezüglich des Chiralitätszentrums $\overset{*}{C}$ in den beiden möglichen R- und S-Konfigurationen sowie als Gemische der daraus resultierenden Diastereomeren vorliegen können, und wobei die Verbindungen der Formel I, bezüglich der Iminogruppe sowohl in der syn-Form als auch in der anti-Form vorliegen können und wobei diese Verbindungen der Formel I auch in den verschiedenen Hydratformen vorliegen können, und die pharmazeutisch verwendbaren Salze dieser Verbindungen der Formel I starke antibakterielle Eigenschaften aufweisen sowie die Eigenschaften besitzen, das Wachstum und die Futterverwertung bei Tieren zu verbessern.

Weiterhin wurde gefunden, daß man die neuen ß-Lactam-Antibiotica der Formel I erhält, wenn man Verbindungen der Formel II

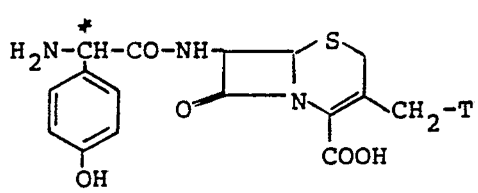

(II)

in welcher

$\overset{*}{C}$ und T die oben angegebene Bedeutung haben, oder deren Salze, mit Verbindungen der Formel III

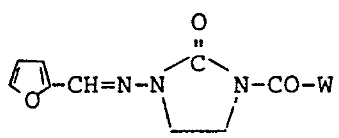

(III)

in welcher

W für Halogen, Azid oder eine andere nukleofuge Abgangsgruppe steht,

in Gegenwart eines Lösungsmittels und gegebenenfalls eines Säurebindemittels bei Temperaturen von etwa -20°C bis etwa +50°C umsetzt und die erhaltenen ß-Lactam-Antibiotica gegebenenfalls in ihre pharmazeutisch verwendbaren Salze überführt oder aus den erhaltenen Salzen gewünschtenfalls die freien Säuren herstellt.

Die erfindungsgemäßen Verbindungen zeigen neben guter antibakterieller Wirksamkeit eine ausgezeichnete Verträglichkeit.

Verwendet man beispielsweise 7-[D-α-Amino-(4-hydroxyphenyl)-acetamido]-3-acetoxymethyl-ceph-3-em-4-carbonsäure und 1-Chlorcarbonyl-3-furfurylidenamino-imidazolidin-2-on als Ausgangsstoffe, so kann der Reaktionsablauf durch das folgende Formelschema wiedergegeben werden:

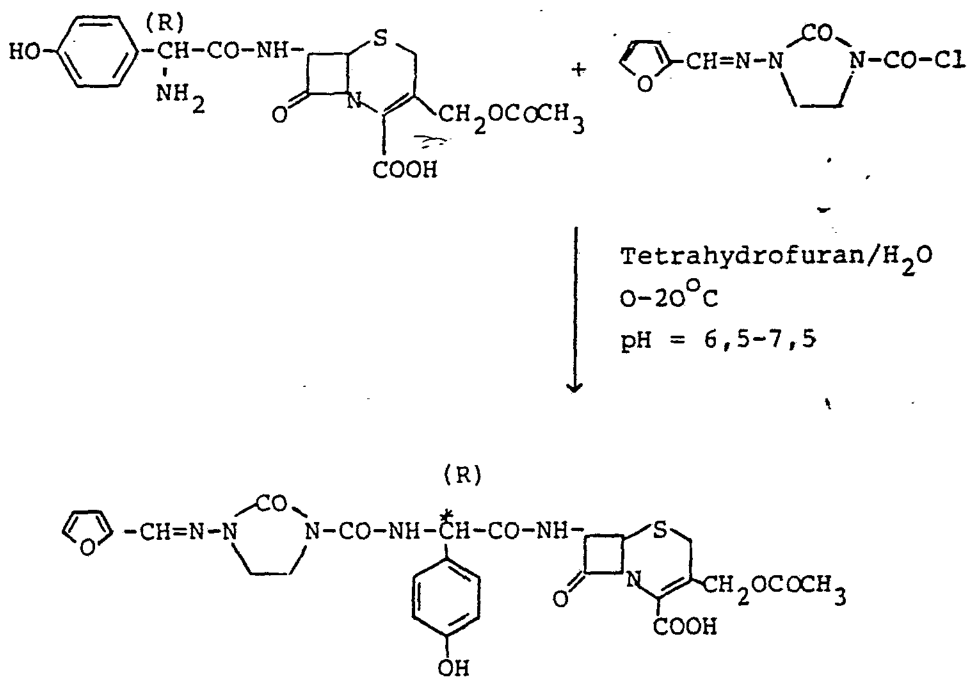


In der Definition von T bedeutet Alkyl in Alkyl-CO-O- vorzugsweise Alkyl mit 1 bis 4, insbesondere 1 oder 2 Kohlenstoffatomen. Beispielhaft seien Methyl und Äthyl genannt, wobei Methyl besonders bevorzugt ist.

Der heterocyclische Ring Het in -S-Het (Definition von T) besteht aus 5 oder 6 Ringgliedern und enthält 1 bis 4, vorzugsweise 1 bis 3 gleiche oder verschiedene Heteroatome, wobei als Heteroatome Sauerstoff, Schwefel und Stickstoff stehen. Bevorzugt ist der heterocyclische Ring ungesättigt und enthält besonders bevorzugt 2 Doppelbindungen. Der heterocyclische Ring kann einen oder mehrere, vorzugsweise 1 oder 2, insbesondere einen Substituenten enthalten. Als Substituenten seien beispielhaft aufgeführt: Halogen, wie Fluor, Chlor und Brom, vorzugsweise Chlor und Brom, Amino, Niederalkylamino, Diniederalkylamino, Niederalkyl, Cycloalkyl (mit 3 bis 7, vorzugsweise 5

oder 6 Kohlenstoffatomen im Cycloalkylteil), Niederalkyloxy, Trifluormethyl, Phenyl, Benzyl und Acylamino mit vorzugsweise 2 bis 5, insbesondere 2 oder 3 Kohlenstoffatomen. Als -S-Het seien als besonders bevorzugt aufgeführt:

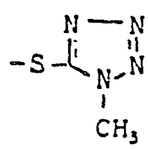 , 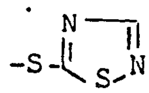 , 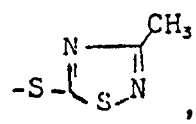 ,

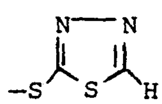 , 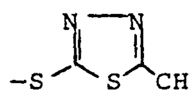 , 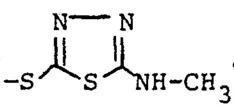 , 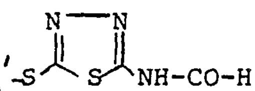 ,

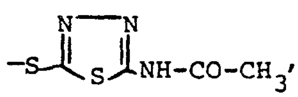 , 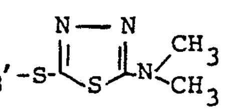 , 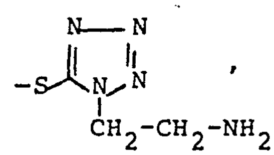 ,

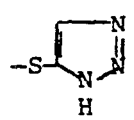 , 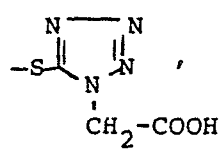 , 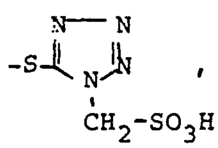 , 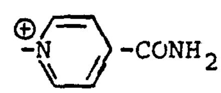

Der -S-Phenylrest in der Definition von T kann einen oder mehrere, vorzugsweise 1 bis 3, insbesondere 1 oder 2 gleiche oder verschiedene Substituenten tragen, wobei als Substituenten diejenigen bevorzugt werden, welche oben als mögliche Substituenten des Restes -S-Het aufgeführt werden.

Ganz besonders bevorzugt sind erfindungsgemäße Verbindungen, in welchen $\overset{*}{C}$ in der R-Konfiguration vorliegt.

Alle Kristallformen und Hydratformen der erfindungsgemäßen Verbindungen der allgemeinen Formel I und ihrer Salze sind in gleicher Weise antibakteriell wirksam.

Halogen W steht für Fluor, Chlor und Brom, vorzugsweise für Brom oder Chlor, insbesondere für Chlor.

Unter nukleofugen Abgangsgruppen in der Definition von W sind alle üblicherweise in der organischen Chemie verwendeten nukleofugen Gruppen und vor allem solche zu verstehen, welche in Angewandte Chemie, 81 (1969), Seite 543 beschrieben sind.

Pharmazeutisch verwendbare Salze der Verbindungen der Formel I sind Salze dieser Verbindungen mit anorganischen und organischen Basen an der sauren Carboxylgruppe beziehungsweise den sauren Carboxyl- und Sulfonsäuregruppen. Als Basen können hierzu alle in der pharmazeutischen Chemie, insbesondere in der Chemie der Antibiotika, üblicherweise verwendeten Basen eingesetzt werden. Als anorganische Basen seien beispielhaft genannt: Alkali- und Erdalkalihydroxide, Alkali- und Erdalkalicarbonate und Alkalihydrogencarbonate, wie Natrium- und Kaliumhydroxid, Calcium- und Magnesiumhydroxid, Natrium- und Kaliumcarbonat, Calciumcarbonat, Natrium- und Kaliumhydrogencarbonat; Aluminiumhydroxid und Ammoniumhydroxid. Als organische Amine können primäre, sekundäre und tertiäre aliphatische Amine sowie heterocyclische Amine eingesetzt werden. Beispielhaft seien genannt: Di- und Triniedrigalkylamine, z. B. Diäthylamin, Triäthylamin, Tri-ß-hydroxyäthylamin, Procain, Dibenzylamin, N,N'-Dibenzyläthylendiamin, N-Benzyl-ß-phenyl-äthylamin, N-Methyl- und N-Äthylmorpholin, 1-Ephenamin, Dehydroabietylamin, N,N'-Bis-dehydroabietyläthylendiamin, N-Niedrigalkylpiperidin. Auch sogenannte basische Aminosäuren wie Lysin oder Arginin können vorteilhaft als Basen Verwendung finden. Besonders bevorzugte Salze sind die Natriumsalze.

Ganz besonders bevorzugte Verbindungen der Formel I sind solche, bei denen

T für einen Rest aus der Gruppe

-H, -OH, $-OCONH_2$, $-OCOCH_3$, 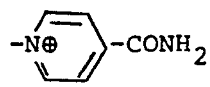

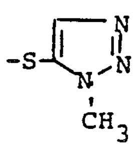 , 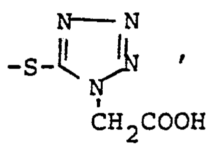 , 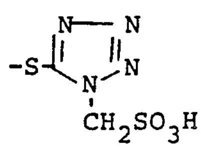 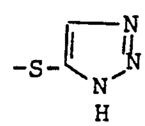

steht und

C* in der R-Konfiguration vorliegt sowie die pharmazeutisch verwendbaren Salze dieser Verbindungen, insbesondere die Natriumsalze.

Die als Ausgangsstoffe verwendeten Verbindungen der allgemeinen Formel II sind bereits bekannt oder nach bekannten Methoden erhältlich.

Alle Kristallformen, Hydratformen und Salze der Verbindungen der allgemeinen Formel II sind als Ausgangsmaterialien für das erfindungsgemäße Verfahren geeignet.

Als Beispiele seien genannt:
7-[α-Amino-(4-hydroxyphenyl)acetamido]-3-methyl-ceph-3-em-4-carbonsäure, 7-[α-Amino-(4-hydroxyphenyl)-acetamido]-3-[(1-methyl-tetrazol-5-yl)-thiomethyl]-ceph-3-em-4-carbonsäure, 7-[α-Amino-(4-hydroxyphenyl)-acetamido]-3-[(1H-1,2,3-triazol-5-yl)-thiomethyl]-ceph-3-em-4-carbonsäure.

Als Salze der Verbindungen der Formel II können vorzugsweise Salze mit Basen eingesetzt werden, welche als für die Salzbildung mit Verbindungen der Formel I geeignet aufgeführt werden. Besonders bevorzugt sind die Natriumsalze.

Die als Ausgangstoffe verwendeten Verbindungen der allgemeinen Formel III sind nach bekannten Methoden erhältlich. Sie können z. B. auf folgendem Wege erhalten werden (vgl. auch J.A.C.S. 78 (1956) 5349):

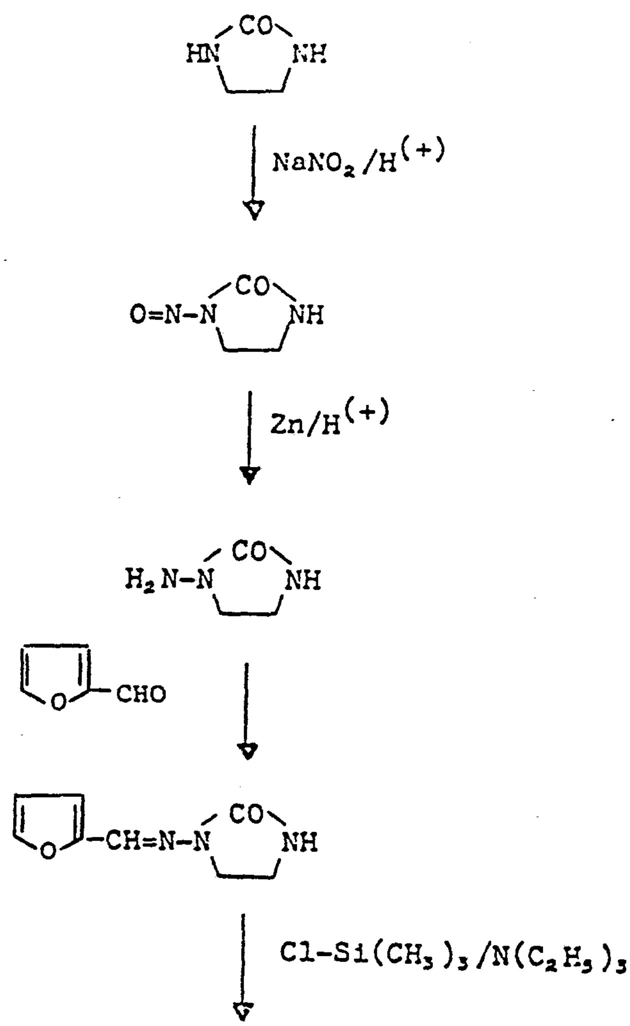

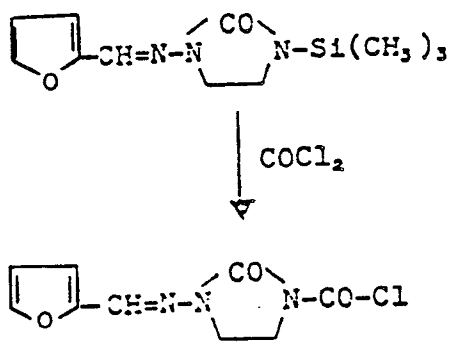

Diejenigen Verbindungen der allgemeinen Formel III, in denen W Azid ist, werden in üblicher Weise z. B. aus den entsprechenden Verbindungen III, in denen W Halogen ist, durch Umsetzung beispielsweise mit Alkaliaziden erhalten.

Als Verdünnungsmittel kommen beim erfindungsgemäßen Verfahren Wasser sowie alle inerten organischen Lösungsmittel, vorzugsweise solche, welche mit Wasser mischbar sind, in Frage. Hierzu gehören vor allem niedere Dialkylketone, z. B. Aceton, Methyläthylketon, cyclische Aether, z.B. Tetrahydrofuran und Dioxan; Nitrile, z.B. Acetonitril; niedere Dialkylformamide, z.B. Dimethylformamid; niedere Alkylalkohole, z.B. Aethanol und Isopropanol sowie Dimethylsulfoxid. Diese Lösungsmittel können auch in Mischungen untereinander sowie in beliebigen Mischungen einzelner oder mehrerer dieser Lösungsmittel mit Wasser verwendet werden. Das erfindungsgemäße Verfahren kann also durchgeführt werden in Gegenwart von: (a) ausschließlich Wasser, (b) ausschließlich einem oder mehreren organischen Lösungsmitteln oder (c) Wasser und einem oder mehreren organischen Lösungsmitteln. Ist wegen des Vorhandenseins von Wasser eine pH-Messung während der erfindungsgemäßen Reaktion möglich, wird der pH der Reaktionsmischung durch Zusatz von Basen oder durch Verwendung von Puffergemischen vorzugsweise zwischen 6,5 bis 7,5 gehalten. Das erfindungsgemäße Ver-



BAD ORIGINAL

fahren läßt sich aber auch sehr gut in einem anderen pH-Bereich, beispielsweise zwischen 4,5 und 9,o oder bei pH 2,o bis 4,5, durchführen. Ferner ist es möglich, die Reaktion in mit Wasser nicht mischbaren Lösungsmitteln, z. B. halogenierten Kohlenwasserstoffen, wie Chloroform oder Methylenchlorid, unter Zusatz von organischen Basen, vorzugsweise Niederalkylaminen, z. B. Triäthylamin, Diäthylamin oder cyclischen Basen, z. B. N-Äthylpiperidin durchzuführen. Weiterhin läßt sich die Reaktion in einer Mischung aus Wasser und einem mit Wasser nicht mischbaren Lösungsmittel, wie z. B. Niederalkyläthern, wie Diäthyläther, halogenierten Kohlenwasserstoffen, wie Chloroform und Methylenchlorid; Schwefelkohlenstoff; Isobutylmethylketon; Estern wie Essigsäureäthylester; aromatischen Kohlenwasserstoffen wie Benzol, ausführen, wobei es zweckmäßig ist, kräftig zu rühren und den pH-Wert durch Basenzusatz oder Verwendung von üblichen Pufferlösungen, z. B. Phosphat-, Acetat- oder Citratpuffer, zwischen 4,5 und 9,o oder z. B. 2,o und 4,5 zu halten. Man kann die Reaktion aber auch in Wasser allein in Abwesenheit von organischen Lösungsmitteln in Gegenwart einer organischen oder anorganischen Base oder unter Zusatz von üblichen Pufferstoffen durchführen.

Als Säurebindemittel können alle in der Chemie der Antibiotica üblicherweise verwendeten Säurebinder verwendet werden. Hierzu gehören anorganische Basen und organische Basen, welche z. B. durch sterische Hinderung schwer acylierbar sind. Als Beispiele für anorganische Basen seien Natrium- und Kaliumhydroxid genannt. Als organische Basen kommen praktisch alle nicht oder schwer acylierbaren offenkettigen oder cyclischen



BAD ORIGINAL

Amine und auch heteroaromatische Basen in Frage. Als Basen seien beispielhaft tertiäre Amine, vorzugsweise Niederalkylamine, z.B. Triäthylamin und/oder cyclische Basen, z.B. Pyridin sowie als schwer acylierbares sekundäres Amin Dicyclohexylamin genannt.

Beim erfindungsgemäßen Verfahren ist der Zusatz einer Base nur dann erforderlich, wenn während der Reaktion saure Verbindungen entstehen, z.B. im Falle, daß W für Halogen oder Azid steht.

Die Reaktionstemperaturen können in einem größeren Bereich variiert werden. Im allgemeinen arbeitet man zwischen etwa -20°C und etwa +50°C, vorzugsweise zwischen 0 und +20°C. Wie bei den meisten chemischen Reaktionen können jedoch prinzipiell auch höhere oder niedrigere Temperaturen verwendet werden.

Die Umsetzung kann bei Normaldruck, aber auch bei vermindertem oder erhöhten Druck ausgeführt werden. Im allgemeinen arbeitet man bei Normaldruck.

Bei der Durchführung der erfindungsgemäßen Verfahren können die Anteile der Reaktionspartner der Formeln II und III in weiten Grenzen variiert werden, ohne daß das Ergebnis nachteilig beeinflußt wird. Die Ausgangsstoffe können z.B. in äquimolekularen Mengen miteinander zur Reaktion gebracht werden. Es kann jedoch zweckmäßig sein, einen der beiden Reaktionspartner im Überschuß zu verwenden, um sich die Reinigung oder Reindarstellung des gewünschten Penicillins zu erleichtern und die Ausbeute zu erhöhen.

Beispielsweise kann man die Reaktionspartner der allgemeinen Formel II mit einem Ueberschuß von 0,1 bis 0,3 Moläquivalenten einsetzen und dadurch eine geringere Zersetzung der Reaktionspartner der allgemeinen Formel III in einem wasserhaltigen Lösungsmittelgemisch erreichen. Der Ueberschuß der Reaktionspartner der allgemeinen Formel II läßt sich wegen der guten Löslichkeit in wäßrigen Mineralsäuren beim Aufarbeiten des Reaktionsgemisches leicht entfernen.

Andererseits kann man aber auch mit Vorteil die Reaktionspartner der allgemeinen Formel III mit einem Ueberschuß von beispielsweise 0,1 bis 1,0 Moläquivalenten einsetzen. Dadurch werden die Reaktionspartner der allgemeinen Formel II besser ausgenützt und die als Nebenreaktion in wasserhaltigen Lösungsmitteln ablaufende Zersetzung der Reaktionsteilnehmer der allgemeinen Formel III kompensiert. Da die im Ueberschuß zugesetzten Verbindungen der allgemeinen Formel III sich in Wasser rasch in neutrale stickstoffhaltige Heterocyclen umwandeln, die sich leicht entfernen lassen, wird die Reinheit der Antibiotica hierdurch kaum beeinträchtigt.

Die Menge der gegebenenfalls verwendeten Basen ist z. B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich ist oder nicht sinnvoll ist, werden vorzugsweise 2 Moläquivalente Base zugesetzt.

Die Aufarbeitung der Reaktionsansätze zur Herstellung der erfindungsgemäßen Verbindungen und ihrer Salze erfolgt durchweg in der bei diesen Körpern allgemein bekannten Art und Weise. Auch die Isolierung und Reinigung der erfindungsgemäßen Verbindungen sowie die Freisetzung der freien Säuren

aus Salzen oder die Umwandlung der freien Säuren in Salze werden nach allgemein üblichen Methoden der organischen Chemie, welche jedem Fachmann geläufig sind, vorgenommen.

Die Verbindungen der allgemeinen Formel I sind in Form der freien Säure sowohl kristallin wie amorph und sowohl wasserfrei wie in verschiedenen Hydratformen in gleicher Weise antibakteriell wirksam. Ebenfalls sind die Verbindungen der allgemeinen Formel I in Form ihrer Salze, z. B. Natriumsalze, sowohl kristallin wie amorph und sowohl wasserfrei wie wasserhaltig, beispielsweise als Hydrat, in gleicher Weise antibakteriell wirksam.

Als neue Wirkstoffe seien beispielhaft genannt (Formel IV):

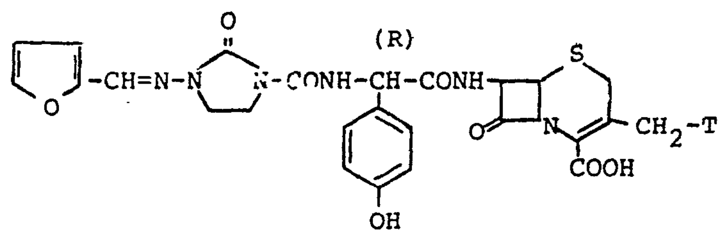

(IV)

| | I |
|---|---|
| IVa | - OH |
| IVb | $- OCOCH_3$ |
| IVc | $- OCONH_2$ |
| IVd | 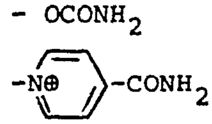 |
| IVe | 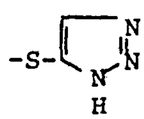 |

IVf

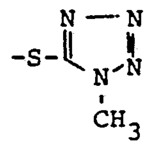

IVg

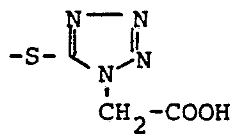

IVh

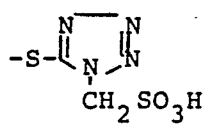

Die erfindungsgemäßen Wirkstoffe weisen bei geringer Toxizität eine starke und breite antimikrobielle Wirksamkeit auf. Diese Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Wirkstoffe sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Wirkstoffe gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph.epidermidis, Staph.aerogenes und Gaffkya tetragena (Staph. = Staphylococcus);

Lactobacteriaceae, wie Streptokokken, z.B. Streptococcus pyogenes, α-bzw. β-hämolysierende Streptokokken, nicht (γ-)-hämolysierende Streptokokken, Str.viridans, Str. faecalis (Enterokokken), Str.agalactiae, Str.lactis, Str.equi, Str.anaerobis und Diplococcus pneumoniae (Pneumokokken) (Str. = Streptococcus);
Neisseriaceae, wie Neisserien, z.B. Neisseria gonorrhoeae (Gonokokken), N.meningitidis (Meningokokken), N.catarrhalis und N.flava (N. = Neisseria);
Corynebacteriaceae, wie Corynebakterien, z.B. Corynebacterium diphtheriae, C.pyogenes, C.diphtheroides, C.acnes, C.parvum, C.bovis, C.renale, C.ovis, C.murisepticum, Listeria-Bakterien, z.B. Listeria monocytogenes, Erysipelothrix-Bakterien, z.B. Erysipelothrix insidiosa, Kurthia-Bakterien, z.B. Kurthia zopfii (C. = Corynebacterium);

Mycobacteriaceae, wie Erreger von Mykobakteriosen, z.B. Mycobacterium tuberculosis, M.bovis, M.avium, sogenannte atypische Mykobakterien der Runyon-Gruppen I, II, III und IV, M.leprae (M. = Mycobacterium);

Enterobacteriaceae, wie Escherichiae-Bakterien der Coli-Gruppe: Escherichia-Bakterien, z.B. Escherichia coli, Enterobacter-Bakterien, z.B. E.aerogenes, E.cloacae, Klebsiella-Bakterien, z.B. K.pneumoniae, K.pneumoniae, K.ozaenae, Erwiniae, z.B. Erwinia spec., Serratia, z.B. Serratia marcescens (E. = Enterobacter) (K. = Klebsiella),

Proteäe-Bakterien der Proteus-Gruppe: Proteus, z.B. Proteus vulgaris, Pr.morganii, Pr.rettgeri, Pr.mirabilis, Providencia, z.B. Providencia sp. (Pr. = Proteus), Salmonelleae: Salmonella-Bakterien, z.B. Salmonella paratyphi A und B, S.typhi, S.enteritidis, S.cholerae suis, S.typhimurium (S. = Salmonella, Shigella-Bakterien, z.B. Shigella dysenteriae, Sh. ambigua, Sh.flexneri, Sh.boydii, Sh.sonnei (Sh. = Shigella);

Pseudomonadaceae, wie Pseudomonas-Bakterien, z.B. Pseudomonas aeruginosa, Ps.pseudomallei (Ps. = Pseudomonas). Aeromonas-Bakterien, z.B. Aeromonas liquefaciens, A. hydrophila (A. = Aeromonas);

Spirillaceae, wie Vibrio-Bakterien, z.B. Vibrio cholerae, V.proteus, V.fetus (V. = Vibrio), Spirillum-Bakterien, z.B. Spirillum minus;

Parvobacteriaceae oder Brucellaceae, wie Pasteurella-Bakterien, z.B. Pasteurella multocida, Past.pestis (Yersinia), Past.pseudotuberculosis, Past.tularensis (Past. = Pasteurella), Brucella-Bakterien, z.B. Brucella abortus, Br.melitensis, Br.suis (Br. = Brucella), Haemophilus-Bakterien, z.B. Haemophilus influenzae, H.ducreyi, H.suis, H.canis, H.aegypitcus (H. = Haemophilus), Bordetella-Bakterien, z.B. Bordetella pertussis, B.bronchiseptica (B. = Bordetella), Moraxella-Bakterien, z.B. Moraxella lacunata;

Bacterioidaceae, wie Bacteroides-Bakterien, z.B. Bacteroides fragilis, B.serpens (B. = Bacteroides), Fusiforme-Bakterien, z.B. Fusobacterium fusiforme, Sphaerophorus-Bakterien, z.B. Sphaerophorus necrophorus, Sph.necroticus, Sph.pyrogenes (Sph. = Sphaerophorus);



BAD ORIGINAL

Bacillaceae, wie aerobe Sporenbildner, z.B. Bacillus anthracis (B.subtilis, B.cereus) B. = Bacillus), Anaerobe Sporenbildner-Chlostridien, z.B. Clostridium perfringens, Cl.septicium, Cl.oedematien, Cl.histolyticum, Cl.tetani, Cl.botulinum (Cl. = Clostridium);

Spirochaetaceae, wie Borrelia-Bakterien, z.B. Borrelia recurrentia, B.vincentii (B. = Borrelia), Treponema-Bakterien, z.B. Treponema pallidum, Tr.pertinue, Tr. carateum (Tr. = Treponema), Leptospira-Bakterien, Leptospira interrogans, z.B. Leptospira icterohaemorrhagiae, L.canicola, L.grippotyphosa, L.pomona, L.mitis, L.bovis (L. = Leptospira);

Die obige Aufzählung von Erregern ist lediglich beispielhaft und keineswegs beschränkend aufzufassen.

Als Krankheiten, die durch die erfindungsgemäßen Wirkstoffe verhindert, gebessert und/oder geheilt werden können, seien beispielsweise genannt:

Erkrankungen der Atmungswege und des Rachenraumes;

Otitis; Pharyngitis; Pneumonie; Peritonitis; Pyelonephritis; Cystitis; Endocarditis; Systeminfektionen; Bronchitis; Arthritis;lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Ueberzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.



BAD ORIGINAL

Lösungen und Emulsionen können neben dem oder den Wirkstoffen die üblichen Trägerstoffe wie Lösungsmittel, Lösungsvermittler und Emulgatoren, z.B. Wasser, Aethylalkohol, Isopropylalkohol, Aethylcarbonat, Aethylacetat, Benzylalkohol, Benzylbenzoat, Propylenglykol, 1,3-Butylenglykol, Dimethylformamid, Oele, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Aethylalkohol, Propylenglykol, Suspendiermittel, z.B. äthoxylierte Isostearylalkohole, Polyoxyäthylensorbit- und Sorbitanester, mikrokristallineCellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5 , vorzugsweise von etwa 0,5 bis 95 Gewichtsprozent der Gesamtmischung vorhanden sein.



BAD ORIGINAL

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 5 bis etwa 1000, vorzugsweise 20 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 10 bis 100 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung



BAD ORIGINAL

und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die neuen Cephalosporine zeichnen sich durch starke antibakterielle Wirkungen, die in vivo und in vitro geprüft wurden, und durch orale Resorbierbarkeit aus.

Die erfindungsgemäßen Cephalosporine können zum Zwecke der Erweiterung des Wirkungsspektrums und um eine Wirkungssteigerung speziell bei ß-lactamasebildenden Bakterien zu erzielen mit anderen antimikrobiellen Wirkstoffen z.B. mit Penicillinen, die besonders penicillinasefest sind, kombiniert werden. Eine solche Kombination wäre z.B. die mit Oxacillin oder Dicloxacillin.

Die erfindungsgemäßen Cephalosporine können zum Zweck der Erweiterung des Wirkungsspektrums und um eine Wirkungssteigerung zu erreichen auch mit Aminoglykosidantibiotica, wie z.B. Gentamicin, Kanamicin, Amikacin oder Tobramicin, kombiniert werden.

In vivo-Versuche

Aus der folgenden Tabelle 1 geht die Wirkung einer der erfindungsgemäßen Verbindungen gegen eine Reihe von Bakterien im Tierversuch mit der weißen Maus hervor. Die weißen Mäuse vom Stamm $CF_1$ wurden intraperitoneal mit der jeweils angegebenen Bakterienart infiziert.

Tabelle 1

Tierversuche mit der weißen Maus

Bestimmung der $ED_{100}$ nach 24 Stunden

| Keim | Dosis in mg des ß-Lactamantibiotikums des Beispiels 1 pro kg/Körpergewicht (subcutan) |
|---|---|
| Escherichia coli C 165 | 2 x 150 |
| Klebsiella 63 | 2 x 150 |

Therapie : zweimalig : 30 und 90 Minuten nach der Infektion. Die $ED_{100}$ ist die Dosis, bei der 100% der infizierten Tiere nach 24 Stunden noch überleben.

Das erfindungsmäße Verfahren sei durch die folgenden Beispiele erläutert:

Bei den NMR-Spektren der erfindungsgemäßen Verbindungen bedeuten die Bezeichnungen in den Klammern:



BAD ORIGINAL

s = Singulett

d = Dublett

dd = Doppeldublett

$A_2B_2$ = $A_2B_2$-System

Erläuterungen der in den Beispielen verwendeten Abkürzungen:

Gew.-Tle. = Gewichtsteile

Vol.-Tle. = Volumenteile

THF = Tetrahydrofuran

Essigester = Essigsäureäthylester

Zers.-p = Zersetzungspunkt

Die Ausbeuteangaben in % bedeuten Ausbeuten in % der Theorie.

Beispiel 1

Natrium-7-{(D-α-[(2-oxo-3-furfurylidenamino-imidazolidin-1-yl)-carbonylamino]-4-hydroxyphenyl-acetamido}-3-acetoxymethyl-ceph-3-em-4-carboxylat

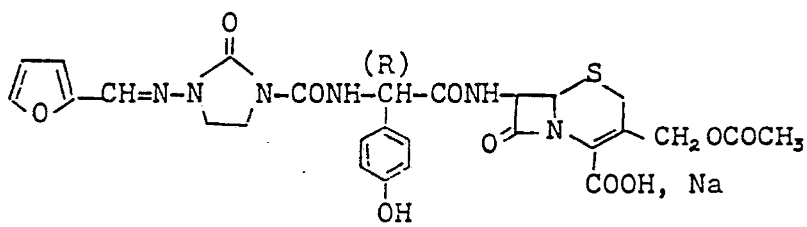

Eine auf 5° gekühlte Lösung von 7,0 Gew.Tln. 7-(D-α-Amino-4-hydroxyphenyl-acetamido)-3-acetoxymethyl-ceph-3-em-4-carbonsäure in 120 Vol.Tln. 80-proz. wäßrigem THF wird mit 1N Natronlauge bis zum konstanten pH von 8 versetzt. Dazu werden portionsweise 3,1 Gew.Tle. 1-Chlorcarbonyl-2-oxo-3-furfurylidenamino-imidazolidin gegeben, währenddessen der pH durch Zugabe von 0,5N Natronlauge auf 7,5 gehalten wird. Wenn keine Lauge mehr verbraucht wird, wird von einer geringen Menge Unlöslichem abgesaugt, 120 Vol.Tle. Wasser zugegeben und das THF schonend abgezogen. Die verbleibende wäßrige Lösung wird einmal mit Essigester extrahiert, auf 5° gekühlt, mit 100 Vol.Tln. eiskaltem Essigester überschichtet und mit 0,5N Salzsäure auf pH 1,8 angesäuert, wobei die Säure ausfällt. Sie wird abgesaugt, einmal mit Essigester gewaschen, auf Ton abgepreßt und im Exsiccator über Phosphorpentoxid und Kaliumhydroxid getrocknet (5,7 Gew.Tle. = 69,9 %). Die Säure wird in 50 Vol.Tln. Wasser (5°) suspendiert und derart tropfenweise mit 0,3N Natronlauge versetzt, daß ein pH-Bereich zwischen 6,0 und 7,5 eingehalten werden kann. Nach der nahezu vollständigen Auflösung der Säure (End-pH 7,5) wird filtriert und die hellbraune Lösung gefriergetrocknet. Man erhält 5,0 Gew.Tle. (59,3 %) mit einem Zers.p. von 235°.

IR(KBr.): 1765, 1725, 1660, 1600, 1415, 1230 $cm^{-1}$

NMR($CD_3OD/D_2O$): s 7.70(1H), d 7.66(1H,I=1Hz), $A_2B_2$ 7.33 und 6.86 (4H) mit überlagertem $H_3$ des Furanringes bei ca. 6.88, dd zentr. 6.56(I=1Hz und 3Hz; 1H), d 5.70(1H), s 5.30(1H); s(breit) 3.90(4H), s 2.10(3H) ppm (δ).
Die übrigen Protonen sind verdeckt vom Lösungsmittelsignal und vom Signal der austauschbaren Protonen.

Der β-Lactamgehalt beträgt 95 % (HPLC).

Beispiel 2

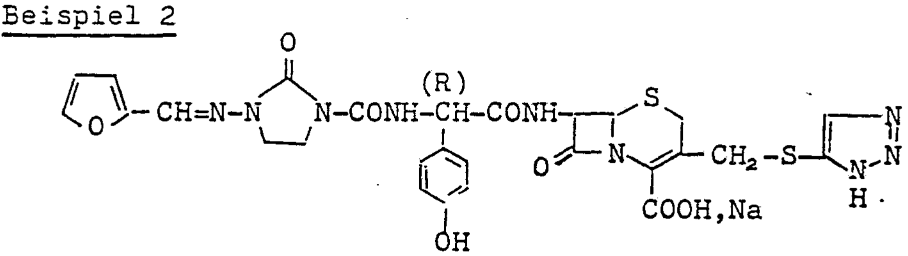

1,0 Gew.Tle. 7-[D-α-Amino-(4-hydroxyphenyl)-acetamido]-3-[(1H-1,2,3-triazol-5-yl)-methylthio]-ceph-3-em-4-carbonsäure (DOS 2 500 386) und 0,6 Gew.Tle. 1-Chlorcarbonyl-2-oxo-3-furfurylidenamino-imidazolidin werden wie in Beispiel 1 umgesetzt und aufgearbeitet. Man erhält 0,9 Gew.Tle. Natrium-7-{D-α-[(2-oxo-3-furfurylidenamino-imidazolidin-1-yl)carbonylamino]-(4-hydroxyphenyl)-acetamido}-3-[1H-1,2,3-triazol-5-yl)-methylthio]-ceph-3-em-4-carboxylat vom Zersp. 230°.

Patentansprüche

1. Verbindungen der Formel (I)

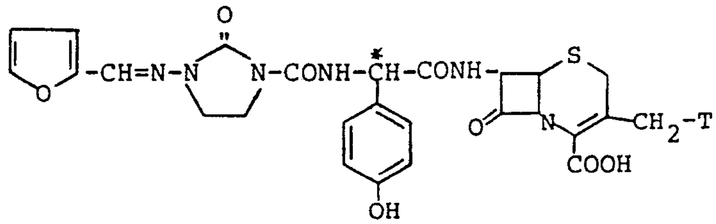

(I)

in der

T Wasserstoff, Alkyl-CO-O-, Pyridinium, 4-Carbamoylpyridinium, Aminopyridinium, Carbamoyloxy, Azido, Cyano, Hydroxy, die Gruppe -S-Phenyl, welche substituiert sein kann, oder die Gruppe -S-Het bedeutet, in welcher Het für einen gegebenenfalls substituierten heterocyclischen 5- oder 6-gliedrigen Ring steht;

wobei diese Verbindungen der Formel I bezüglich des Chiralitätszentrums C* in den beiden möglichen R- und S-Konfigurationen sowie als Gemische der daraus resultierenden Diastereomeren vorliegen können, und wobei die Verbindungen der Formel I, bezüglich der Iminogruppe sowohl in der syn-Form als auch in der anti-Form vorliegen können und wobei diese Verbindungen der Formel I auch in den verschiedenen Hydratformen vorliegen können, und die pharmazeutisch verwendbaren Salze dieser Verbindungen.

2. Verbindungen gemäß Anspruch 1, bei denen T einen Rest aus der Gruppe

-H, -OH, $-OCONH_2$, $-OCOCH_3$ 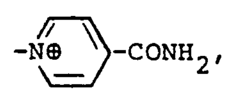,

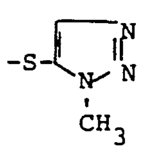

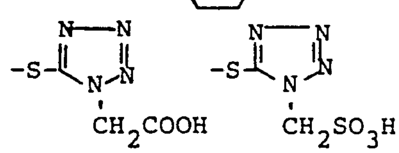

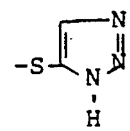

bedeutet und
$\overset{+}{C}$ in der R-Konfiguration vorliegt.

3. Verbindung der Formel

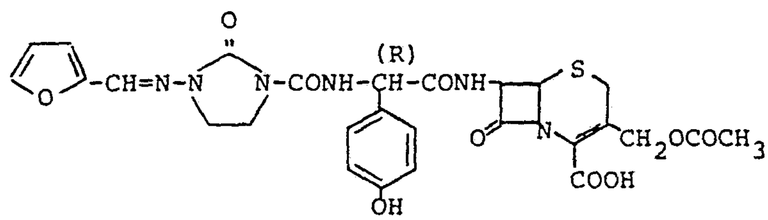

sowie ihre pharmazeutisch verwendbaren Salze.

4. Verbindung der Formel

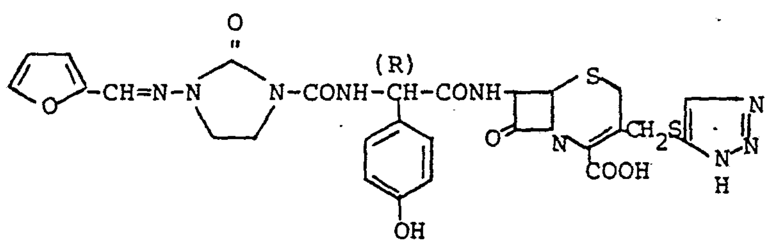

sowie ihre pharmazeutisch verwendbaren Salze.

5. Die Natriumsalze der Verbindungen gemäß den Ansprüchen 1 bis 4.

6. Verfahren zur Herstellung von Verbindungen der Formel I, dadurch gekennzeichnet, daß man Verbindungen der Formel II

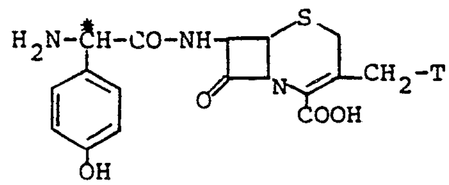

(II)

in welcher

$\overset{*}{C}$ und T die oben angegebene Bedeutung haben, oder deren Salze mit Verbindungen der Formel III

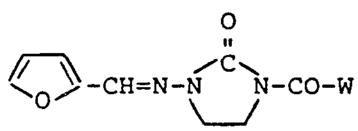

(III)

in welcher

W für Halogen, Azid oder eine andere nukleofuge Abgangsgruppe steht,

in Gegenwart eines Lösungsmittels und gegebenenfalls eines Säurebindemittels bei Temperaturen von etwa $-20^{\circ}C$ bis etwa $+50^{\circ}C$ umsetzt und die erhaltenen ß-Lactam-Antibiotica gegebenenfalls in ihre pharmazeutisch verwendbaren Salze überführt oder aus den erhaltenen Salzen gewünschtenfalls die freien Säuren herstellt.

7. Arzneimittel, gekennzeichnet durch einen Gehalt an einer Verbindung gemäß den Ansprüchen 1 bis 5.

8. Verfahren zur Herstellung eines Arzneimittels, dadurch gekennzeichnet, daß man eine Verbindung gemäß den Ansprüchen 1 bis 5 mit pharmazeutisch geeigneten Träger- und/oder Zusatzstoffen vermischt.

9. Verfahren zur Behandlung von durch Bakterien hervorgerufenen Erkrankungen, dadurch gekennzeichnet, daß man Verbindungen gemäß den Ansprüchen 1 bis 5 Menschen oder Tieren appliziert, die an diesen Erkrankungen leiden.

10. Verwendung von Verbindungen gemäß den Ansprüchen 1 bis 5 bei der Bekämpfung von Erkrankungen.

11. Futterzusatzmittel, gekennzeichnet durch einen Gehalt an einer Verbindung gemäß den Ansprüchen 1 bis 5.

12. Verwendung von Verbindungen gemäß den Ansprüchen 1 bis 5 zur Förderung des Wachstums und zur Verbesserung der Verwertung des Futters bei Tieren.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 78 10 0356

0000393

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 525 541 (BAYER)<br>* Seiten 1-12; Seiten 72-73; Beispiel 8.4; Seiten 135-318; Beispiele 32-35 *<br>-- | 1-12 |
| E | FR - A - 2 359 145 (BAYER)<br>* Seiten 42-47; Seite 38, Beispiel 7 *<br>-- | 1-12 |
| P | DE - A - 2 658 718 (BAYER)<br>* Seiten 158-164; Patentansprüche *<br>-- | 1-12 |
| | DE - A - 2 456 307 (BAYER)<br>* Seiten 68-78 *<br>-- | 1-12 |
| | DE - A - 2 512 998 (BAYER)<br>* Seiten 115-126 *<br>---- | 1-12 |

KLASSIFIKATION DER ANMELDUNG (Int.Cl.²)

C 07 D 501/20
A 61 K 31/545
A 23 K 1/17

RECHERCHIERTE SACHGEBIETE (Int. Cl.²)

C 07 D 501/20

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8,11,12
Unvollständig recherchierte Patentansprüche:
Nicht recherchierte Patentansprüche: 9-10
Grund für die Beschränkung der Recherche: Verfahren zur chirurgischen oder therapeutischen Behandlung des menschlichen oder tierischen Körpers.

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 04-10-1978 | LUYTEN |